# EUROPEAN PATENT APPLICATION

(11) **EP 3 128 058 A1**
(43) Date of publication of application: **08.02.2017**
(21) Application number: 15773470.8
(22) Date of filing: 09.03.2015
(51) Int. Cl.: D04H 1/495

(54) **NONWOVEN FABRIC**

(30) Priority: 04.04.2014 JP 2014078206
(71) Applicant: Unicharm Corporation, Ehime 799-0111 (JP)
(72) Inventor: KIMURA, Akihiro, Kanonji-shi Kagawa 769-1602 (JP); DETANI, Ko, Kanonji-shi Kagawa 769-1602 (JP); KAMEDA, Noritomo, Kanonji-shi Kagawa 769-1602 (JP)
(74) Representative: Peter, Julian
(86) International application number: PCT/JP2015/056780
(87) International publication number: WO 2015/151727

(57) **Abstract**

The present invention pertains to a nonwoven fabric that can effectively allow the passage of high-viscosity waste. The nonwoven fabric (1) is formed from a substrate (10), which extends in an approximately planar shape and has a first surface (FF) and a second surface (FS) positioned at the reverse side from the first surface, and a plurality of protrusions (12), which protrude from the substrate to the first surface side. The fiber density of the first surface side is greater than the fiber density of the second surface side at the apices of the protrusions.

## Description

### Technical Field

The present invention pertains to a nonwoven fabric in which a highly viscous waste can effectively permeate therethrough.

### Background Art

Patent Literature 1 discloses a nonwoven fabric including first projection portions projecting to the first surface side which is on the side observed in planar view of the sheet-like nonwoven fabric, and second projection portions projecting to the second surface side which is on the opposite side to the first surface, the first projection portions and the second projection portions alternately extending in two directions of the first direction and the second direction in planer view of the nonwoven fabric, and the fiber density in the first surface side at the apex portion of each of the first projection portions being lower than the fiber density in the second surface side thereof.

### Citation List

### Patent Literature

PTL 1: Japanese Unexamined Patent Publication No. 2012-144835

### Summary of Invention

### Technical Problem

However, the nonwoven fabric according to the invention disclosed in Patent Literature 1 may cause clogging of a highly viscous waste, such as the soft feces of babies in age of lower months, etc., in between the first surface side and the second surface side, due to the fiber density distribution. Accordingly, it is difficult for the highly viscous waste to effectively permeate through the nonwoven fabric.

The object of the present invention is therefore providing a nonwoven fabric in which a highly viscous waste can effectively permeate therethrough.

### Solution to Problem

To solve the above described problem, the present invention provides a nonwoven fabric including a first surface and a second surface,
the nonwoven fabric including a base portion which extends in an approximately planar shape, and a plurality of protruding portions which protrude from the base portion to a first surface side, wherein
at an apex portion of each of the protruding portions, a fiber density in the first surface side is higher than a fiber density in a second surface side.

### Advantageous Effects of Invention

According to the nonwoven fabric of the present invention, a highly viscous waste can effectively permeate therethrough, by virtue of its fiber density.

### Brief Description of Drawing

Fig. 1 is a planar view of a nonwoven fabric according to an embodiment of the present invention.
Fig. 2 is a partial end surface view along a cross-section II-II of FIG. 1.
Fig. 3 is a photo of a cross-section in which portion III of FIG. 2 is enlarged.
Fig. 4 is a photo of a cross-section in which portion IV of FIG. 2 is enlarged.
Fig. 5 is a schematic view showing the general outline of manufacturing equipment to manufacture the nonwoven fabric according to the embodiment of the present invention.
Fig. 6 is an enlarged view of portion VI in FIG. 5.
Fig. 7 is a partial schematic perspective view of a nonwoven fabric according to comparative examples 1 and 2.

### Description of Embodiments

### (Embodiment)

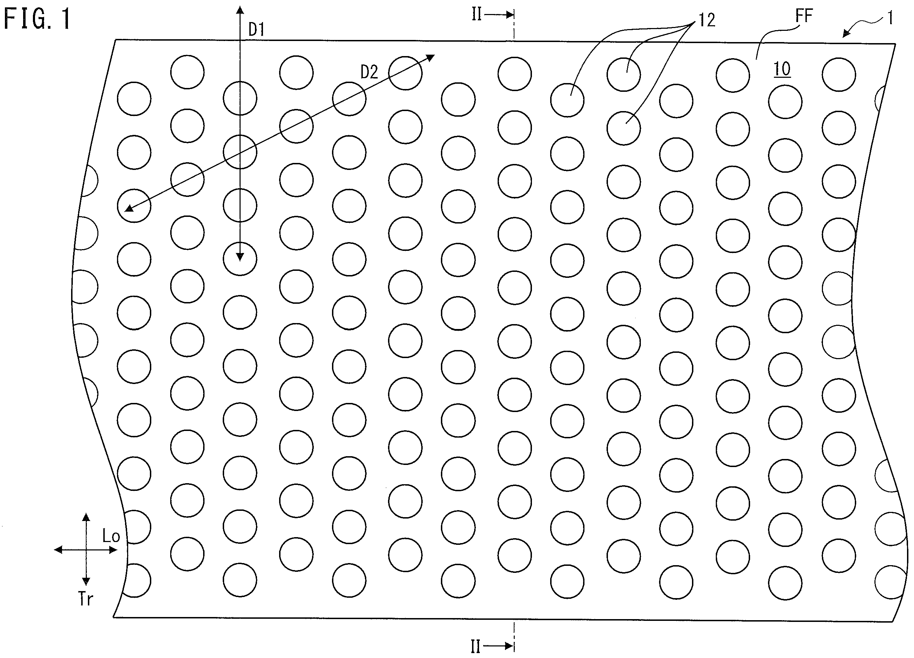

Hereinbelow, a nonwoven fabric 1 according to an embodiment of the present invention is described with reference to FIG. 1 to FIG. 4.

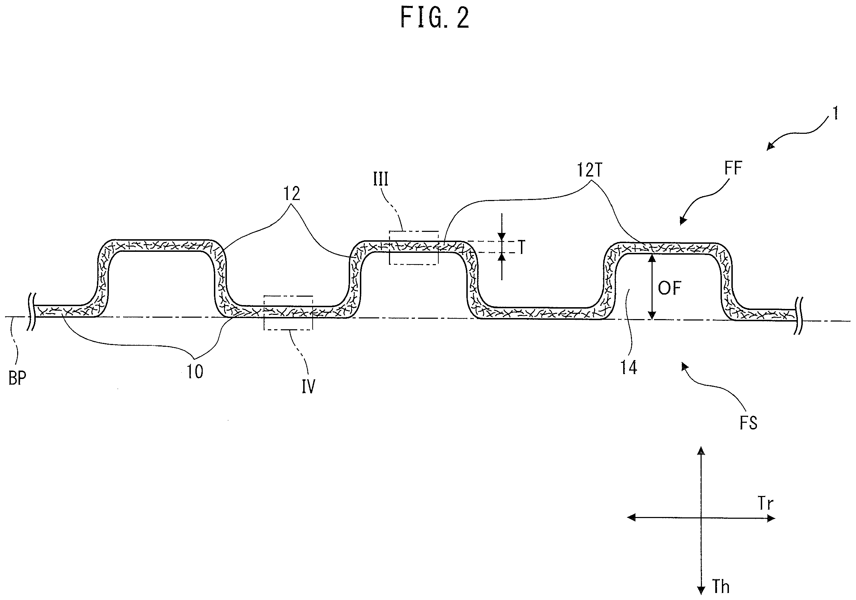

Fig. 1 is a planar view of the nonwoven fabric 1 according to the embodiment, and Fig. 2 is a partial end surface view along a cross-section II-II of FIG. 1. The nonwoven fabric 1 according to the embodiment extends in a plane defined by a longitudinal direction Lo and a transverse direction Tr, and has the first surface FF which can be observed in a planar view of FIG. 1 and a second surface FS which is positioned at the opposite side of the first surface FF.

Referring to FIG. 1 and FIG. 2, the nonwoven fabric 1 is formed by a base portion 10 which extends in an approximately planar shape, and a plurality of protruding portions 12 which protrude from the base portion 10 to the side of the first surface FF. Each of the protruding portions 12 includes an apex portion 12T which is most protruded from the base portion 12, in other words, which is most distant from the base portion 10 in a thickness direction Th of the nonwoven fabric 1.

In the present embodiment, each of the protruding portions 12 has an approximately cylinder-like shape in appearance. In another embodiment, each of the protruding portions 12 may take the shape of, for example a conic shape, a truncated cone, an elliptic or a polygonal cylinder, an elliptic or a polygonal conic shape or an elliptic or a polygonal truncated cone, etc. In still another embodiment, each of the protruding portions 12 may take a hemisphere shape.

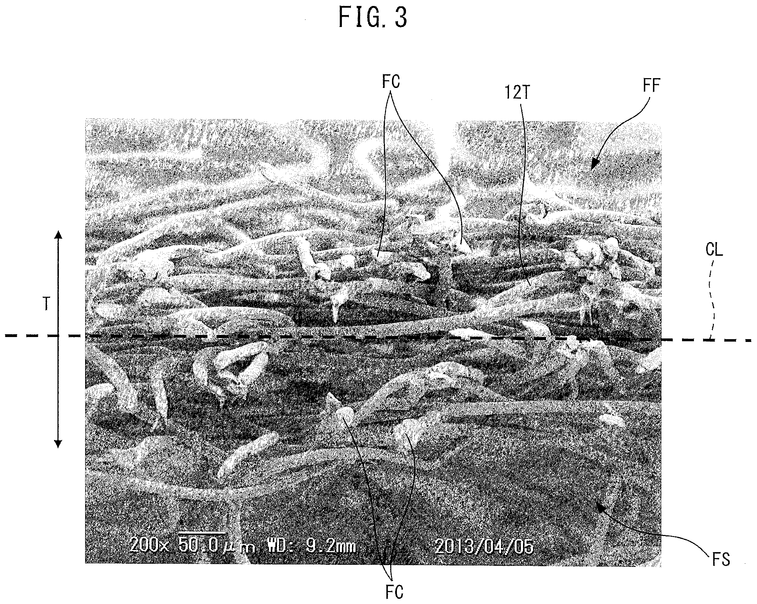

Fig. 3 is a photo of a cross-section in which portion III of FIG. 2 is enlarged. Referring to FIG. 3, at the apex portion 12T of each of the protruding portions 12 in the nonwoven fabric 1 according to the present embodiment, the fiber density in the first surface FF side is higher than the fiber density in a second surface FS side.

In the present invention, "the fiber density" uses an index of the number of portions FC at which fibers are cut per 1mm², in the cutting plane of the nonwoven fabric 1. To be more specific, the cutting plane of a certain area (for example, approximately 0.5mm²) is observed by using an electron scanning microscope (for example, "Real Surface View Microscope VE-7800" manufactured by Keyence Corporation), and the number of portions FC at which the fibers are cut is counted. Then, the number of cut portions is converted to the number of portions per 1mm², and thus the converted number is obtained as the index of "the fiber density".

Incidentally, in the present invention, "the fiber density in the first surface side" is referred to as the fiber density in the cutting plane positioned on the side closer to the first surface FF than a center line CL, when the nonwoven fabric 1 is divided by the center line CL extending at the midpoint of the thickness T of the nonwoven fabric 1. Further, "the fiber density in the second surface side" is referred to as the fiber density in the cutting plane positioned on the side closer to the second surface FS than the center line CL.

Further, the fibers used in the present embodiment are fibers having a sheath-core structure, in which the material of the sheath is high density polyethylene (HDPE), and the material of the core is polyethylene terephthalate (PET).

The fibers used in the nonwoven fabric according to the present invention may be natural fibers (wool, cotton, etc.), regenerated fibers (rayon, acetate, etc.), thermoplastic resin fibers (polyolefins such as polyethylene, polypropylene, polybutylene, ethylene-vinyl acetate copolymer, ethylene-ethyl acrylate copolymer, ethylene-acrylic acid copolymer, or ionomer resin; polyesters such as polyethylene terephthalate, polybutylene terephthalate, polytrimethylene terephthalate, or polylactic acid; polyamides such as nylon, etc.) or their surface modified fibers. Among these fibers, the thermoplastic resin fibers or their surface modified fibers are preferable. Further, these fibers may be composite fibers such as sheath-core type fibers, side-by-side type fibers, island-sea type fibers; hollow type fibers; atypical fibers such as flat, Y-type or C-type fibers; solid crimp fibers such as latent crimped or actual crimped fibers; split fibers split by physical load by water flow, heat or embossing; etc. Incidentally, these fibers may be hydrophilic fibers, or may be hydrophobic fibers. However, when using hydrophobic fibers, an application of hydrophilic oil solution to the fibers, etc., are additionally required.

Hereinbelow, the function of the nonwoven fabric 1 according to the present embodiment is described. Here, as a representative application example of the nonwoven fabric 1 according to the present embodiment, using a top sheet of an absorbent article such as a disposable diaper or a sanitary napkin, by disposing the first surface FF on the skin facing side of a wearer, and disposing the second surface FS on the absorbent body side may be cited. Hereinbelow, the function achieved by the nonwoven fabric 1 used in the above described representative application example is described.
(1) In the absorbent article according to the above described representative application example, the base portion 10 of the nonwoven fabric 1 abuts the absorbent body. At this time, since the base portion 10 of the nonwoven fabric 1 extends in an approximately planar shape, the area abutting the absorbent body is broader, compared to the nonwoven fabric having the projection portions which project also to the second surface side and abut the absorbent body as disclosed in Patent Literature 1. Accordingly, the nonwoven fabric 1 can easily allow the highly viscous waste which has permeated through the base portion 10 to migrate into the absorbent body.
(2) In the absorbent article according to the above described representative application example, the apex portion 12T of each of the protruding portions 12 directly abuts the skin surface of a wearer when wearing the absorbent article. At this time, since the fiber density in the first surface FF side is higher than the fiber density in the second surface FS side, at the apex portion 12T in the nonwoven fabric 1 according to the present embodiment, the easiness of permeation by the capillary action is decreased in the direction from the first surface FF to the second surface FS, and it becomes more difficult for the highly viscous waste to permeate therethrough, whereby the highly viscous waste can be migrated to portions other than the apex portion 12T, or in other words, to the base portion 10. As a result, the apex portion 12T of each of the protruding portions 12 directly abutting the skin surface of the wearer can prevent the highly viscous waste from remaining, whereby it becomes difficult for the highly viscous waste to be attached again to the wearer's skin.
(3) In the absorbent article according to the above described representative application example, the apex portion 12T of each of the protruding portions 12 directly abuts the skin surface of a wearer. At this time, since the fiber density in the first surface FF side is higher than the fiber density in the second surface FS side, at the apex portion 12T of each of the protruding portions 12 in the nonwoven fabric 1 according to the present embodiment, the surface of the apex portion 12T of each of the protruding portions 12 directly abutting the skin surface of the wearer is smooth having less roughness, and provides a comfortable texture.

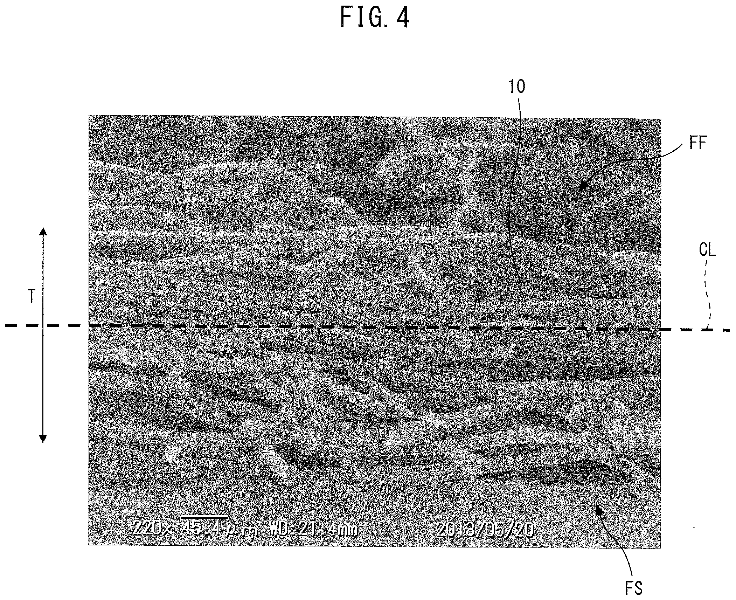

Fig. 4 is a photo of a cross-section in which portion IV of FIG. 2 is enlarged. In the nonwoven fabric 1 according to the present embodiment, at the base portion 10, the fiber density in the first surface FF side is lower than the fiber density in the second surface FS side. By including such configuration, when the nonwoven fabric 1 is used in the absorbent article according to the above described representative application example, the highly viscous waste excreted to the first surface FF of the base portion 10 can easily permeate from the first surface FF to the second surface FS side. As a result, when the nonwoven fabric 1 is used in the absorbent article according to the above described representative application example, the amount of the highly viscous waste remaining in the first surface FF side which is positioned at the skin facing side can be reduced. Accordingly, the highly viscous waste can further be prevented from attaching again to the wearer's skin.

In the nonwoven fabric 1 according to another embodiment, at the base portion 10, the fiber density in the first surface FF side is the same as, or is higher than the fiber density in the second surface FS side.

Further, in the nonwoven fabric 1 according to the present embodiment, the fiber density in the first surface FF side at the apex portion 12T of each of the protruding portions 12 is higher than the fiber density in the first surface FF side at the base portion 10. By including such configuration, when the nonwoven fabric 1 is used in the absorbent article according to the above described representative application example, it is difficult for the apex portion 12T of each of the protruding portions 12 to absorb the highly viscous waste excreted to the first surface FF of the nonwoven fabric 1, whereby it becomes easier for the highly viscous waste to flow into the base portion 10. As a result, the apex portion 12T of each of the protruding portions 12 can prevent the highly viscous waste from remaining, and also the base portion 10 can allow the highly viscous waste to effectively permeate therethrough. Accordingly, the highly viscous waste can further be prevented from attaching again to the wearer's skin.

In the nonwoven fabric according to another embodiment, the fiber density in the first surface FF side at the apex portion 12T of each of the protruding portions 12 is the same as, or is lower than the fiber density in the first surface FF side at the base portion 10.

Further, referring to FIG. 2, in the nonwoven fabric 1 according to the present embodiment, the second surface FS at the apex portion 12T of each of the protruding portions 12 is dented from a virtual planar surface BP in which the second surface FS at the base portion 10 extends toward the first surface FF side. To be more specific, the second surface FS at the apex portion 12T of each of the protruding portions 12 is dented from the planar surface BP by OF [mm] in the thickness direction Th of the nonwoven fabric 1. By such distance, a dented portion 14 is formed on the second surface FS side at portions where the protruding portions 12 are formed on the first surface FF side. As a result, when the nonwoven fabric 1 is used in the absorbent article according to the above described representative application example, the highly viscous waste which has permeated from the first surface FF side to the second surface FS side can be stocked in the space formed by the dented portion 14.

In another embodiment, the nonwoven fabric is formed in such a manner that the planar surface BP in which the second surface FS at the base portion 10 extends and the second surface FS at the apex portion 12T of each of the protruding portions 12 are of substantially the same planar surface.

Further, referring to FIG. 1, in the nonwoven fabric 1 according to the present embodiment, the protruding portions 12 are aligned along a first direction D1 and along a second direction D2. Incidentally, these first direction D1 and second direction D2 may be in any direction. In the present embodiment, the first direction D1 is the same as the transverse direction Tr, and the second direction D2 is tilted from the first direction D1 by 60°. By disposing the protruding portions 12 in this manner, the protruding portions 12 can be disposed in the nonwoven fabric 1 with a preferable distribution for the highly viscous waste to permeate therethrough.

In another embodiment, the protruding portions 12 are aligned along either one of the first direction D1 and the second direction D2. In still another embodiment, the protruding portions 12 are not aligned along any direction.

Further, referring to FIG. 1, in the nonwoven fabric 1 according to the present embodiment, each of the protruding portions 12 is intermittently arranged in the first direction D1 and in the second direction D2, with the base portion 10 disposed in between. It can be understood that the neighboring protruding portions 12 both in the first direction D1 and in the second direction D2 are respectively arranged with the base portion 10 disposed in between. As a result, the highly viscous waste excreted to the first surface FF can be migrated from the apex portion 12T of each of the protruding portions 12 to the neighboring base portion 10, and also the highly viscous waste can further be prevented from attaching again to the wearer's skin.

In another embodiment, the protruding portions 12 are arranged continuously and integrally both in the first direction D1 and in the second direction D2. That is to say, the base portion 10 is intermittently arranged with each of the protruding portions 12 disposed in between, in the same manner as the protruding portions 12 in the nonwoven fabric 1 according to the present embodiment.

In the above, cases in which the nonwoven fabric 1 according to the present embodiment is used in the absorbent article according to the above described representative application example have been described. However, in an absorbent article of another application example, as a separate body from the top sheet, the nonwoven fabric 1 according to the present embodiment is partially adhered onto the top sheet.

Further, in still another application example of the nonwoven fabric 1 according to the present embodiment, the first surface FF is disposed on the absorbent body side, the second surface FS is disposed on the skin facing side of the wearer, and the nonwoven fabric 1 is used as a top sheet of an absorbent article. In this case, the positional relationship between the base portion and the protruding portions are reversed, and the roles thereof are also reversed. That is to say, at the base portion, the fiber density in the second surface FS side is higher than the fiber density in the first surface FF side, and at the protruding portions, the fiber density in the second surface FS side is lower than the fiber density in the first surface FF side. These characteristics are advantageous in allowing the highly viscous waste to permeate from the second surface FS side to the first surface FF side, when the highly viscous waste is excreted to the second surface FS.

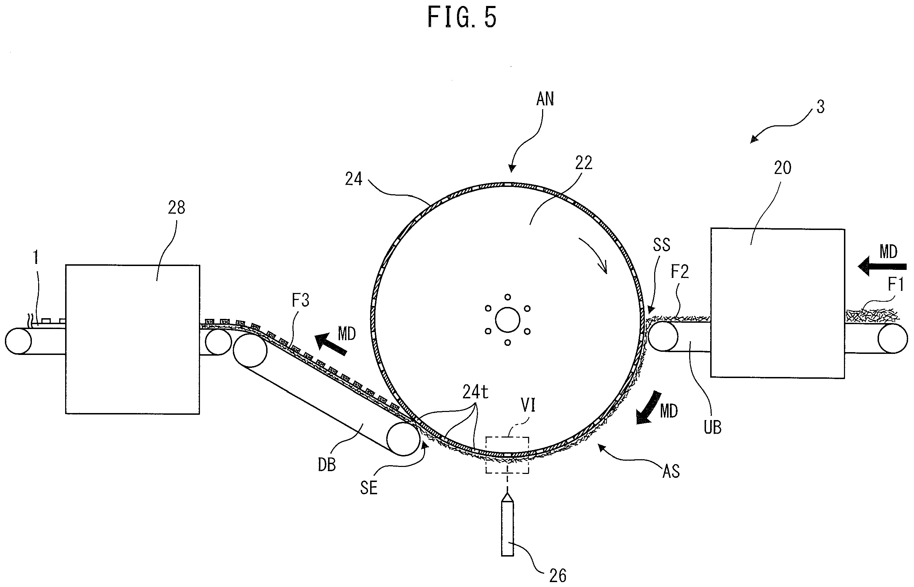

Hereinbelow, the manufacturing method of the nonwoven fabric 1 according to the present embodiment is described. Fig. 5 is a schematic view showing the general outline of the manufacturing equipment 3 to manufacture the nonwoven fabric 1 according to the embodiment of the present invention. The manufacturing equipment 3 is provided with a carding machine 20 which opens and adjusts the basis weight of the fibers F1, a suction drum 22 and an air jet nozzle 26 which form the shape of the nonwoven fabric 1 into the fibers F2, and a heat processing machine 28 which performs heat processing for the fiber F3 so that the shape formed into the fibers F3 is fixed. Incidentally, in FIG. 5, the later described fibers F1 to F3 and the nonwoven fabric 1 are conveyed in the direction shown in the arrow MD, and the conveying direction MD matches with the longitudinal direction Lo of the nonwoven fabric 1.

Referring to FIG. 5, in the manufacturing steps of the nonwoven fabric 1 according to the present embodiment, the opened fibers F1 is first supplied to the carding machine 20. In the carding machine 20, the fibers F1 is further opened, and the basis weight (mass per unit area) of the fibers F1 is adjusted to a desirable value.

The fibers F2 having passed through the carding machine 20 is then supplied to the suction drum 22. The interior of the suction drum 22 is formed to be hollow, and the interior of the suction drum 22 has negative pressure, due to the air being sucked by suction means such as a blower, etc. A plurality of suction holes are provided on the exterior surface of the suction drum 22 (which are not shown), whereby the outside air can be sucked. Incidentally, the size of the suction holes of the suction drum 22 is very small, and thus the fibers F2 are not sucked into the interior of the suction drum 22.

The exterior surface of the suction drum 22 is covered by a pattern plate 24 in the entire circumference thereof, and to be more specific, the fibers F2 are supplied onto the pattern plate 24. In the present embodiment, the pattern plate 24 is a punching plate in which through holes 24t each having a complementary shape to each of the protruding portions 12 of the nonwoven fabric 1 are provided with the distribution of the protruding portions 12.

According to such configuration, the suction holes of the suction drum 22 exposed at the through holes 24t of the pattern plate 24 suck the fibers F2 supplied onto the pattern plate 24. Incidentally, in the nonwoven fabric 1 according to the present embodiment, the positional difference of the height of the first surface FF in the thickness direction Th of the nonwoven fabric 1 between the base portion 10 and the apex portion 12T of each of the protruding portions 12 is approximately equal to the thickness of the pattern plate 24.

Incidentally, in the present embodiment, the suction drum 22 is configured so that on the exterior surface thereof, the suction is performed within the area AS from the point SS at which the fibers F2 are passed from the upstream belt conveyor UB to the point SE at which the fibers F2 are passed on to the downstream belt conveyor DB, and the suction is not performed in the other areas AN. Such configuration is adopted so as to improve the efficiency of the suction function by the suction drum 22.

The fibers F2 sucked onto the exterior surface of the suction drum 22 are blown with warm air by the air jet nozzle 26. Here, the air jet nozzle 26 has a mechanism which uniformly jets a predetermined amount of the warm air in a uniform width in the width direction. By adjusting the width of the blowing ports, the distance between the blowing ports and the fibers F2, etc., the air jet nozzle 26 is configured so that the warm air is substantially uniformly jetted over the entire width of the laminate formed by the fibers F2. The nonwoven fabric 1 according to the present embodiment can be formed into the shape of the fibers F2by the suction function and the jetting function by the suction drum 22 and the air jet nozzle 26.

The temperature of the warm air jetted from the air jet nozzle 26 is higher than the melting point of the fibers F2, however, the temperature of the warm air is adjusted not to be too high, in order to prevent the nonwoven fabric 1 from being too stiff after the nonwoven fabric 1 is completed. Further, the velocity of the warm air is determined so as to form the fibers F2 into a desired shape. Generally, the temperature and the velocity of the warm air jetted from the air jet nozzle 26 differ according to the material of the fibers to be used, the basis weight, the shape of the nonwoven fabric 1 after completion, etc. However, the optimal temperature and velocity are preferably determined for example by experiments, etc. In the present embodiment, the temperature of the warm air jetted from the air jet nozzle 26 is 180 [°C], and the velocity thereof is 45 [m/sec]. For example, the temperature of the warm air jetted from the air jet nozzle 26 ranges preferably from 80 [°C] to 400 [°C], and the velocity thereof ranges preferably from 10 to 200 [m/sec]. Incidentally, at this stage, the fibers F2 can be formed, and at the same time, the shape thereof can be fixed to a certain degree, by jetting the warm air to the fibers F2 with a temperature higher than the melting point thereof.

Incidentally, in the manufacturing equipment 3 according to the present embodiment, the surface of the laminate formed by the fibers F2 facing the suction drum 22 and the pattern plate 24 is to be the first surface FF of the nonwoven fabric 1, and the surface of the laminate facing the air jet nozzle 26 is to be the second surface FS of the nonwoven fabric 1.

Further, at the portions of the fibers F2 corresponding to the apex portion 12T of each of the protruding portions 12 in the nonwoven fabric 1, the fibers move toward the suction drum 22 in the thickness direction of the fibers F2, by the suction function of the suction drum 22 and by the jetting function of the air jet nozzle 26. By such movement of the fibers, in the nonwoven fabric 1 according to the present embodiment, as described above, at the apex portion 12T of each of the protruding portions 12, the fiber density in the first surface FF side is higher than the fiber density in the second surface FS side.

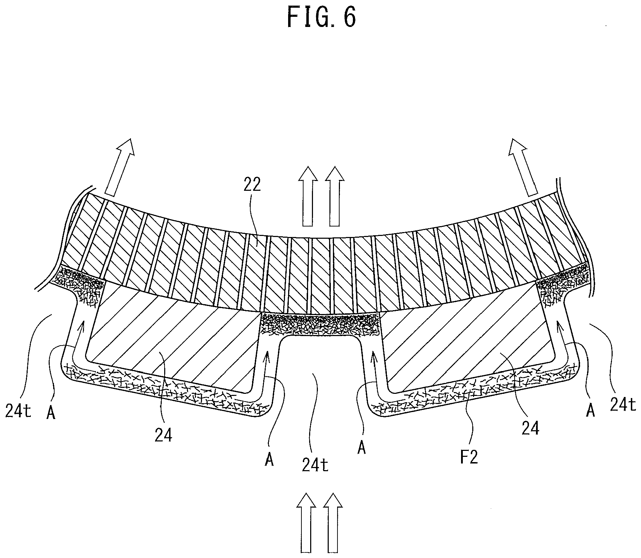

Fig. 6 is an enlarged view of portion VI in FIG. 5. At the portions corresponding to the base portion 10 of the nonwoven fabric 1, in the laminate formed by the fibers F2, the fibers positioned at the pattern plate 24 side in the thickness direction of the fibers F2 in the laminate are sucked toward the exterior surface of the suction drum 22 via the through holes 24t of the pattern plate 24 positioned in the vicinity thereof, whereby move in the direction shown by arrow A in FIG. 6. By such movement of the fibers, as described above, in the nonwoven fabric 1 according to the present embodiment, at the base portion 10, the fiber density in the first surface FF side is lower than the fiber density in the second surface FS side.

Further, the shape of the protruding portions 12 is determined by the shape of the through holes 24t of the pattern plate 24, the velocity of the warm air jetted from the air jet nozzle 26, etc.

Incidentally, by mainly reducing the velocity of the warm air jetted from the air jet nozzle 26, or adjusting the suction from the suction drum 22, the jetting from the air jet nozzle 26, the basis weight of the fibers F2, etc., the second surface FS of the nonwoven fabric 1 can be formed only with the protruding portions 12, without the dented portion 14.

Referring again to FIG. 5, the fibers F3 formed by the above described suction and jetting functions are then transferred to the heat processing machine 28. Fibers F3 are subjected to heat processing in the heat processing machine 28, and the shape formed in the prior stages is fixed. In the heat processing machine 28, by performing the heat processing for the fibers F3 at a relatively low temperature with respect to the melting point of the fibers and with the warm air of low velocity for long hours, the shape of the fibers F3 formed at the prior stages are fixed, and can also provide flexibility to the nonwoven fabric 1. Generally, the temperature and the velocity of the warm air in the heat processing machine 28, the processing time, etc., differ according to the material of the fibers to be used, the basis weight, etc. However, the optimal temperature and velocity are preferably determined for example by experiments, etc.

When the heat processing of the fibers F3 by the heat processing machine 28 is terminated, the nonwoven fabric 1 is completed. The completed nonwoven fabric 1 is cut to a desired size, and is used.

### Examples

In the present examples, tests for surface remaining feces and surface smoothness based on the specific volume were performed with respect to the nonwoven fabrics set with various conditions.

Tests for surface remaining feces and surface smoothness were performed to samples of the nonwoven fabrics having various design parameters (shape, specific volume, fineness, etc.) which are cut into the size of 100 [mm] x 100 [mm]. Incidentally, the fibers used in the present examples are the fibers having the sheath-core structure, in which the material of the sheath is high density polyethylene (HDPE), and the material of the core is polyethylene terephthalate (PET), in the same manner as in the above described embodiment.

First, the nonwoven fabrics according to the Examples and the Comparative Examples tested in the present examples are described.

### (Example 1)

The nonwoven fabric according to Example 1 is the nonwoven fabric manufactured by the above described manufacturing method, and having the shape shown in FIG. 1 and FIG. 2. The thickness of the pattern plate 24 used in manufacturing the nonwoven fabric according to Example 1 is 2 [mm]. In this pattern plate 24, a plurality of circular through holes 24h having a diameter of 10 mm are formed, and the through holes 24h are provided with a 12 mm interval in the first direction (the transverse direction) and in the second direction (the direction tilted from the transverse direction by 60°). Further, in the same manner as in the above described embodiment, each of the blowing ports of the air jet nozzle 26 has a slit shape, and the width of the slit is 1.0 [mm]. Incidentally, in the same manner as in the above described embodiment, the temperature of the warm air jetted from the air jet nozzle 26 is 180 [°C], and the velocity thereof is 45 [m/sec]. In the nonwoven fabric according to Example 1, at the apex portion 12T of each of the protruding portions, the fiber density in the first surface side is higher than the fiber density in the second surface side. That is to say, the nonwoven fabric according to Example 1 is within the technical scope of the present invention.

### (Examples 2 and 3)

In the nonwoven fabrics according to Examples 2 and 3, at the apex portion 12T of each of the protruding portions, the fiber density in the first surface side is higher than the fiber density in the second surface side, in the same manner as in the nonwoven fabric according to Example 1. That is to say, the nonwoven fabrics according to Examples 2 and 3 are also within the technical scope of the present invention. However, the nonwoven fabric according to Example 2 is different from the nonwoven fabric according to Example 1, in that the diameter of the through holes 24h of the pattern plate 24 used at the time of manufacturing is 5 [mm]. Further, the nonwoven fabric according to Example 3 is different from the nonwoven fabric according to Example 1, in that the temperature of the warm air jetted from the air jet nozzle 26 is 160 [°C], and the velocity thereof is 20 [m/sec]. Still further, the nonwoven fabrics according to Examples 2 and 3 are different from the nonwoven fabric according to Example 1 in the design parameters, as described in the later described Table 1, due to the differences in the manufacturing method.

### (Comparative Examples 1 and 2)

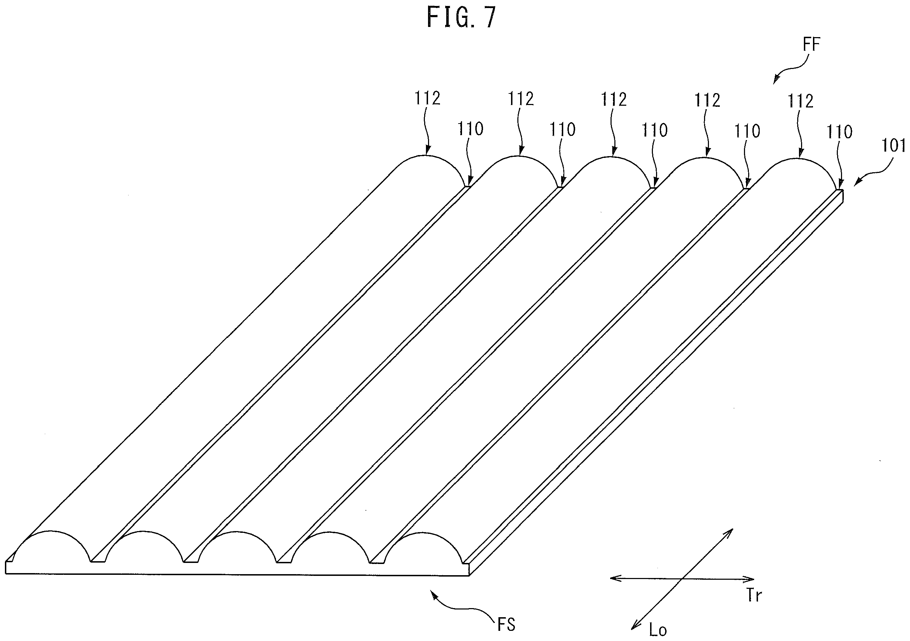

The nonwoven fabrics according to Comparative Examples 1 and 2 are the nonwoven fabrics, in which the base portions 110 and the protruding portions 112 are both formed continuously in the longitudinal direction Lo and are repeatedly formed alternately in the transverse direction Tr, as shown in FIG. 7. The base portions 110 are formed with a 5 mm interval in the transverse direction Tr. The protruding portions 112 are protruded toward the first surface FF side, and are approximately flat on the second surface FS. Incidentally, when manufacturing the nonwoven fabrics according to Comparative Examples 1 and 2, the pattern plate is not used, and by using an air jet nozzle having the blowing ports of a spot shape, air is jetted along the longitudinal direction Lo of the nonwoven fabric at portions corresponding to the base portions 110, to jet and move the fibers to the portions corresponding to the protruding portions 112, whereby the nonwoven fabric having the pattern shown in FIG. 7 is manufactured. By such a manufacturing method, at each of the protruding portions 112, the fiber density in the first surface side is lower than the fiber density in the second surface side. That is to say, the nonwoven fabrics according to Comparative Examples 1 and 2 are not within the technical scope of the present invention. Further, the nonwoven fabrics according to Comparative Examples 1 and 2 are different from the nonwoven fabric according to Example 1 in the design parameters, as described in the later described Table 1, due to the differences in the manufacturing method.

### (Comparative Example 3)

The manufacturing method of the nonwoven fabric according to Comparative Example 3 is different from that of the nonwoven fabric according to Example 1, in that the used punching plate is different. In the punching plate used when manufacturing the nonwoven fabric according to Comparative Example 3, projection portions having a conic shape, the tip of which are rounded, and projecting from the surface by 5 mm are inserted to a part of the through holes 24h of the punching plate used when manufacturing the nonwoven fabric according to Example 1. The projection portions are provided along the above described first direction and along the second direction, so that the through holes 24h and the projection portions are disposed alternately. Accordingly, the nonwoven fabric according to Comparative Example 3 is the nonwoven fabric in which the projection portions are projected toward both sides of the first surface and the second surface, in the same manner as in the nonwoven fabric disclosed in Patent Literature 1. Incidentally, in the nonwoven fabric according to Comparative Example 3, the projection portions projecting toward the first surface side is regarded as the protruding portions. By such a manufacturing method, at each of the protruding portions, the fiber density in the first surface side is lower than the fiber density in the second surface side. That is to say, the nonwoven fabric according to Comparative Example 3 is not within the technical scope of the present invention. Further, the nonwoven fabric according to Comparative Example 3 is different from the nonwoven fabric according to Example 1 in the design parameters, as described in the later described Table 1, due to the differences in the manufacturing method.

### (Comparative Examples 4 to 6)

The nonwoven fabrics according to Comparative Examples 4 to 6 are general air-through nonwoven fabrics having a planer shape, obtained by subjecting the fibers opened by a carding machine set with the design parameters as described in the later described Table 1 to the heat processing.

Next, the methods of the tests performed in the present examples are described. Incidentally, the fiber density is as above described.

### (Specific volume)

The specific volume is a value obtained by dividing the average value of the thickness of the sample at the protruding portions measured under the pressure of 3 gf/cm² three times, by the basis weight of the sample obtained by using the average value of the weight measurement performed three times.

### (Surface remaining feces)

In the measurement of the surface remaining feces, an absorbent body to be used in an absorbent article is placed on a planar surface, and a lamination onto which the samples cut into the size of 100 [mm] x 100 [mm] is pasted is used. In the measurement, first, 3cc of simulated soft feces with the viscosity of 0. 3 Pa·s is dropped onto the first surface of the samples, and is left for five minutes. After five minutes, filter paper is placed on the samples and is pressed by the pressure of 0.36 gf/cm² for 30 seconds. The value obtained by subtracting the weight of the filter paper before pressure from the weight of the filter paper after pressure at this time, and then dividing the subtracted value by the area of the diffused simulated soft feces is set as the index of the surface remaining feces. This index shows that the lower the index, the more the simulated soft feces passes through the samples, and the more effectively the samples allow the highly viscous waste to permeate therethrough. Incidentally, the simulated soft feces is liquid composed by the following composition which has high viscosity (unit being mass %).
Ion exchanged water: 86.4
Sodium chloride (NaCl): 1.0
Glycerin: 10.0
Sodium salt of carboxymethyl cellulose (NaCMC): 1.0
Triton -X: 0.05
Pigment (Red No. 102): 0.05
Powdered cellulose: 1.5

### (Surface smoothness)

In the present examples, the measurement of the surface smoothness is performed by using the automatic surface testing machine (KES-FB4-AUTO-A) manufactured by KATO TEC CO., LTD. The samples are placed on a horizontal measuring table, one end portion of which is mechanically fixed thereto, and the other end portion of which is fixed by a weight placed thereto with a tension of 2 gf/mm so that the samples do not sag during the measurement. As the friction block, the one in which ten piano wires each having the diameter of 0.5 mm configured to be aligned in parallel and have a width of 5 mm is used. At this time, a weight is placed onto the friction block, and the load of the friction block is 50 g. In the measurement, the friction block is horizontally moved for 20 mm in the speed of 1 mm/s, in the two directions of the longitudinal direction Lo (corresponding to the conveying direction MD at the time of manufacturing) and the transverse direction Tr of the nonwoven fabric 1. The variation of the friction coefficient during the measurement (MMD (mean deviation of friction coefficient)) is measured three times for each direction, and the average value thereof is set as the index of the surface smoothness in each measuring direction. This index shows that the lower the index is, the smaller the variation of the friction resistance is, and thus the smoother the surface of the samples is.

Table 1 shows the design parameters of each sample and the test results.

Incidentally, regarding the fiber density in Table 1, fiber density a1 represents the fiber density on the side closer to the first surface than the center line CL at the apex portion 12T in each of the protruding portions, fiber density a2 represents the fiber density on the side closer to the second surface than the center line CL at the apex portion 12T in each of the protruding portions, fiber density b1 represents the fiber density on the side closer to the first surface than the center line CL at the base portion 10, and fiber density b2 represents the fiber density on the side closer to the second surface than the center line CL at the base portion 10. Further, regarding the surface smoothness, Lo and Tr respectively represent the measurement results, when the nonwoven fabric is measured in the longitudinal direction Lo and in the transverse direction Tr.

**[Table 1]**

| Example | | Example 1 | Example 2 | Example 3 | Comparative Example 1 | Comparative Example 2 | Comparative Example 3 | Comparative Example 4 | Comparative Example 5 | Comparative Example 6 |
|---|---|---|---|---|---|---|---|---|---|---|
| Basis Weight (g/m²) | | 25 | 30 | 20 | 35 | 22 | 30 | 25 | 20 | 22 |
| Thickness (mm) | | 1.3 | 1.7 | 2.4 | 2.3 | 1.2 | 2.2 | 1.0 | 1.0 | 1.4 |
| Specific Volume (cm³/g) | | 53.6 | 57.3 | 117.5 | 65.7 | 52.3 | 73.3 | 38.0 | 50.3 | 64.5 |
| Fineness (dtex) | | 1.3 | 1.3 | 1.3 | 3.3 | 1.3 | 1.3 | 2.0 | 3.2 | 4.4 |
| Fiber Density | a1 | 137 | 152 | 143 | 13 | 25 | 21 | | | |
| | a2 | 98 | 105 | 112 | 22 | 32 | 36 | | | |
| | b1 | 57 | 68 | 56 | 29 | 29 | 21 | | | |
| | b2 | 133 | 109 | 85 | 75 | 61 | 59 | | | |
| Surface Remaining Feces (g/m²) | | 91.1 | 82.4 | 36.0 | 189.5 | 632.7 | 136.2 | 846.5 | 673.0 | 375.0 |
| Surface Smoothness | Lo | 0.59 | 0.68 | 0.77 | 0.83 | 0.70 | 0.88 | 0.64 | 0.68 | 1.00 |
| | Tr | 0.63 | 0.69 | 0.88 | 1.09 | 0.92 | 1.13 | 0.86 | 0.87 | 1.00 |

Incidentally, the specific volume is a dominant parameter with respect to the indices of the surface remaining feces and the surface smoothness. Generally, the higher the specific volume is, the lower the value of the surface remaining feces becomes, that is to say, the more tendency there is to improve the performance of the surface remaining feces. This is because when the specific volume is high, more space is formed inside the nonwoven fabric. Further, generally, the lower the specific volume is the lower the value of the surface smoothness becomes, that is to say, the more tendency there is to improve the performance of the surface smoothness. This is because, when the specific volume is low, the fibers are laminated densely, whereby the variation of the friction resistance becomes small.

However, referring to Table 1, when Examples 1 and 2 are compared to Comparative Examples 1 to 3, although the nonwoven fabrics according to Examples 1 and 2, which are within the technical scope of the present invention, include those with lower specific volume compared to those according to the Comparative Examples 1 to 3, the nonwoven fabrics according to Examples 1 and 2 are superior to those according to the Comparative Examples 1 to 3 in the surface remaining feces. Further, the nonwoven fabrics according to Examples 1 and 2 are superior to the nonwoven fabrics according to the Comparative Examples 1 to 3 in the surface smoothness, naturally when the specific volume is lower, and even when the specific volume is equivalent (for example when Example 1 and Comparative Example 2 are compared). Further, from another view point, although the basis weight of the nonwoven fabrics according to Example 2 and Comparative Example 3 is the same, that of Example 2 is superior to that of Comparative Example 3 both in the surface remaining feces and the surface smoothness. Still further, the surface remaining feces can further be reduced by setting the specific volume to an even higher value as in Example 3.

Further, when Examples 1 to 3 and Comparative Examples 4 to 6 are compared, although the nonwoven fabrics according to Examples 1 to 3, which are within the technical scope of the present invention, include those with lower specific volume compared to those according to the Comparative Examples 4 to 6, the nonwoven fabrics according to Examples 1 to 3 are superior to those according to the Comparative Examples 4 to 6 in the surface remaining feces. Further, the nonwoven fabrics according to Examples 1 and 2 are superior to the nonwoven fabrics according to the Comparative Examples 4 to 6 in the surface smoothness, naturally when the specific volume is lower, and even when the specific volume is equivalent (for example when Example 1 and Comparative Example 5 are compared).

All the features which can be understood by those skilled in the art from the description of the specification, drawings and the claims can be applied independently or can be applied in optional combination with another one or a plurality of features disclosed herein to be bound together, as long as such features are explicitly excluded or its technical aspect becomes an impossible or a meaningless combination, even when such features are described only in combination with another specific feature in this specification.

The present invention is defined as follows.
(1) A nonwoven fabric including a first surface and a second surface positioned opposite to the first surface,
   the nonwoven fabric including a base portion which extends in an approximately planar shape, and a plurality of protruding portions which protrude from the base portion to a first surface side, wherein
   at an apex portion of each of the protruding portions, a fiber density in the first surface side is higher than a fiber density in a second surface side.
(2) The nonwoven fabric according to (1), wherein
   at the base portion, a fiber density in the first surface side is lower than a fiber density in the second surface side.
(3) The nonwoven fabric according to (1) or (2), wherein
   the fiber density in the first surface side at the apex portion of each of the protruding portions is higher than the fiber density in the first surface side at the base portion.
(4) The nonwoven fabric according to any one of (1) to (3), wherein
   the second surface at the apex portion of each of the protruding portions is dented from a planer surface in which the second surface at the base portion extends toward the first surface side.
(5) The nonwoven fabric according to any one of (1) to (4), wherein
   the protruding portions are aligned along a first direction and along a second direction.
(6) The nonwoven fabric according to (5), wherein
   each of the protruding portions is intermittently arranged in the first direction and in the second direction, with the base portion disposed in between.

### Reference Signs List

- 1: nonwoven fabric
- 10: base portion
- 12: protruding portion
- 12T: apex portion
- FF: first surface
- FS: second surface

## Claims

1. A nonwoven fabric including a first surface and a second surface positioned opposite to the first surface,
the nonwoven fabric comprising a base portion which extends in an approximately planar shape, and a plurality of protruding portions which protrude from the base portion to a first surface side, wherein
at an apex portion of each of the protruding portions, a fiber density in the first surface side is higher than a fiber density in a second surface side.

2. The nonwoven fabric according to claim 1, wherein
at the base portion, a fiber density in the first surface side is lower than a fiber density in the second surface side.

3. The nonwoven fabric according to claim 1 or 2, wherein
the fiber density in the first surface side at the apex portion of each of the protruding portions is higher than the fiber density in the first surface side at the base portion.

4. The nonwoven fabric according to any one of claims 1 to 3, wherein
the second surface at the apex portion of each of the protruding portions is dented from a planer surface in which the second surface at the base portion extends toward the first surface side.

5. The nonwoven fabric according to any one of claims 1 to 4, wherein
the protruding portions are aligned along a first direction and along a second direction.

6. The nonwoven fabric according to claim 5, wherein
each of the protruding portions is intermittently arranged in the first direction and in the second direction, with the base portion disposed in between.
